# EUROPEAN PATENT APPLICATION

(11) **EP 1 872 784 A1**
(43) Date of publication of application: **02.01.2008**
(21) Application number: 05734445.9
(22) Date of filing: 20.04.2005
(51) Int. Cl.: A61K 31/4535, A61K 9/70, A61K 47/14, A61K 47/16

(54) **MEDICINAL PREPARATION FOR PERCUTANEOUS ABSORPTION**

(71) Applicant: Nichiban Company Limited, Bunkyo-ku, Tokyo 112-8663 (JP); Senju Pharmaceutical Co., Ltd., Osaka-shi, Osaka 541-0046 (JP)
(72) Inventor: KAWAHARA, Kouji, Nichiban Co., Ltd., Tokyo 112-8663 (JP); ARAMOMI, Yasuhiko, Nichiban Co., Ltd., Tokyo 112-8663 (JP); SHIMADA, Noriko, Nichiban Co., Ltd., Tokyo 112-8663 (JP); OHTORI, Akira, Kobe-shi, Hyogo 651-2273 (JP); ISOWAKI, Akiharu, Akashi-shi, Hyogo 674-0061 (JP)
(74) Representative: HOFFMANN EITLE
(86) International application number: PCT/JP2005/007530
(87) International publication number: WO 2006/114868

(57) **Abstract**

The present invention provides a percutaneous absorption formulation including a patch having an adhesive layer disposed on a substrate and the adhesive layer contains ketotifen fumarate and tris(hydroxymethyl) aminomethane, and the patch is packaged in a hygroscopic packaging material. In the percutaneous absorption formulation, tris(hydroxymethyl)aminomethane particularly selected from various basic substances is incorporated, and by packaging the patch in a hygroscopic packaging material, the percutaneous absorptivity and content stability of a drug can be simultaneously improved and the yellowing of the drug can be suppressed. These effects can be further improved by the incorporation of propyl gallate, the use of an adhesive layer including an SIS-based adhesive base and a rosin ester-based adhesion imparting resin, and/or the removal of oxygen from the atmosphere in the inside of the packaging material.

## Description

### TECHNICAL FIELD

The present invention relates to a percutaneous absorption formulation containing as an active ingredient, ketotifen fumarate and having satisfactory percutaneous absorptivity of the active ingredient, and furthermore a drug content in the formulation is stable with time and the yellowing of the formulation is suppressed. More specifically, the present invention relates to a percutaneous absorption formulation including a patch having an adhesive layer containing besides ketotifen fumarate, a specific basic substance disposed on a substrate, and the patch is packaged in a hygroscopic packaging material.

### BACKGROUND ART

It is known that 4-(1-methyl-4-piperidilidene)-4H-benzo[4,5]cyclohepta[1,2-b]tiophene-10(9H)-one (hereinafter, referred to as ketotifen) and it's salts thereof have wide ranging anti-allergy action and anti-histamine action, and are effective for bronchial asthma, allergic rhinitis, eczema, dermatitis, urticaria, pruritus and eye diseases, for example.

Further, the formulation containing as an active ingredient, ketotifen is used in various dosage forms, such as tablets, capsules, syrup, cream, gel and eye-drops. Among them, an oral agent, such as tablets, capsules and syrup is excellent in persistence of a drug efficacy, however has such a problem that side effects, such as drowsiness, gastrointestinal diseases, hepatic dysfunction are exhibited. On the other hand, with respect to cream and gel, it is difficult to maintain a use amount and applied area thereof constant, so that it is lacking in the quantification needed for a systemic treatment and further, a disadvantage is caused wherein at an applied part of human skin may feel greasy and the agents are attached to cloths. Further, with respect to an eye drop, although it is excellent in immediate effectivity, a disadvantage is caused wherein it is likely to be flowed away with a tear and it has poor persistency of a drug efficacy. On the contrary, by a patch, not only a certain amount of a drug can be absorbed by the skin, but also an accidental ingestion and a failure to ingest are unlikely to be caused and further, in the case where a side effect is exhibited, the patch can be immediately peeled off. Therefore, the patch is a dosage form having extremely high usefulness.

When ketotifen fumarate, which is a salt between fumaric acid and ketotifen, is administered through percutaneous absorption, by adding a basic substance, generally ketotifen is isolated as a free base to improve skin permeability of ketotifen. However, although ketotifen as a free base has satisfactory skin permeability, when it is incorporated in a patch, it is dissolved and the drug content is decreased with time, so that it is lacking in content stability, and further it is known that the yellowing of the drug is observed.

For improving the content stability of ketotifen fumarate, it is known that in a therapeutic agent for dermatological diseases, such as dermatitis and eczema, antioxidants, such as hydrogen sulfites, sulfites, pyrosulfites and dibutylhydroxytoluene (BHT) are incorporated (see for example, Patent Document 1). However, with respect to sodium hydrogen sulfite, although it is effective in maintaining the content stability and in the suppression of the yellowing, it has strong irritating odor, and BHT has a problem in the safety in terms of skin irritating.

Further, for maintaining the patch in a stable state for a long period, the patch is usually packaged in a packaging material in which an aluminum foil capable of blocking water, oxygen or light from the outside is laminated, for example. However, with respect to a patch containing as an active ingredient, ketotifen fumarate, even when the patch is packaged with such a packaging material, the content stability of ketotifen fumarate is not improved and also, the yellowing cannot be suppressed. [Patent Document 1] Japanese Patent Application Publication No. JP-A-6-48935

### DISCLOSURE OF THE INVENTION

### Problems to Be Solved by the Invention

Taking such disadvantages into consideration, the present invention has been completed not only to improve simultaneously the percutaneous absorptivity and the content stability of a drug in a patch containing as an active ingredient, ketotifen fumarate, but also to suppress the coloring (yellowing) thereof.

### Means for Solving the Problems

The present inventors have made extensive and intensive studies towards solving the above-noted problems with respect to a patch containing as an active ingredient, ketotifen fumarate. As a result thereof, it has been found that by incorporating tris(hydroxymethyl) aminomethane (hereinafter, referred to as Tris) in an adhesive layer and by packaging the patch in a hygroscopic packaging material, not only the percutaneous absorptivity of a drug becomes improved and the content of a drug with time becomes stable, but also the yellowing is suppressed. Further, it has been also found that by incorporating propyl gallate, by using an SIS-based adhesive base and a rosin ester-based adhesion imparting resin, and by substantially removing oxygen from an atmosphere in the packaging material, the effects of stabilizing the content and suppressing the yellowing can be further enhanced. Based on these novel findings, the present invention has been completed.

Thus, the present invention relates to: a percutaneous absorption formulation including a patch having an adhesive layer disposed on a substrate and the adhesive layer includes ketotifen fumarate and tris(hydroxymethyl)aminomethane, and the patch is packaged in a hygroscopic packaging material.

Preferred embodiments of the present invention are as follows:
the percutaneous absorption formulation in which the adhesive layer further contains 0.01 to 10% by weight of propyl gallate;
the percutaneous absorption formulation in which the adhesive layer includes an SIS-based adhesive base and a rosin ester-based adhesion imparting resin;
the percutaneous absorption formulation in which the adhesive layer includes an SIS-based adhesive base and a rosin ester-based adhesion imparting resin, and further contains 0.01 to 10% by weight of propyl gallate; and
the percutaneous absorption formulation, in which the packaging material has a deoxidation function, the packaging material is oxygen impermeable and a deoxidizing agent is packaged together therein, or the inside of the packaging material is purged with nitrogen.

### Effects of the Invention

According to the present invention, by selecting particularly tris(hydroxymethyl)aminomethane from various basic substances and by incorporating it in a patch containing as an active ingredient, ketotifen fumarate, and further by packaging the patch in a hygroscopic packaging material, the percutaneous absorptivity and content stability of a drug can be simultaneously improved and the yellowing of the drug can be suppressed. Further, at least one of by incorporating propyl gallate, by using an adhesive layer including an SIS-based adhesive base and a rosin ester-based adhesion imparting resin, and by substantially removing oxygen from an atmosphere in the inside of the packaging material through, for example, purging with nitrogen, the above-noted effects can be improved.

### BEST MODES FOR CARRYING OUT THE INVENTION

A patch included in a percutaneous absorption formulation according to the present invention has an adhesive layer containing an adhesive which is disposed on a substrate, and the adhesive layer contains as essential ingredients, ketotifen fumarate and tris(hydroxymethyl)aminomethane, as well as if desired other additives. For the purpose of protecting the adhesive layer until the patch is used, further a release liner also may be disposed on the adhesive layer.

As the adhesive, an adhesive exhibiting pressure-sensitive adhesiveness at normal temperature is used and examples thereof include an acryl-based adhesive, a rubber-based adhesive and a silicone-based adhesive. Among them, an acryl-based adhesive and a rubber-based adhesive are preferred. Particularly a rubber-based adhesive is preferred, because adhesion imparting resins and other additives as a component thereof can be freely selected.

Examples of the rubber-based adhesive include those including a rubbery elastomer as an adhesive base, such as a natural rubber (cis-1,4-isoprene), a synthetic rubber (trans-1,4-isoprene), a styrene-isoprene-styrene block copolymer (SIS), polyisobutylene, polybutene and polyisoprene. Then, to these adhesive bases, added is an adhesion imparting resin, such as a rosin-based resin, for example rosin and rosin derivatives (hydrogenated products, disproportionated products, polymers, esterified products); a terpene resin, for example an α-pinene and β-pinene; a terpene phenol resin; aliphatic, aromatic, alicyclic and copolymeric petroleum resins; an alkyl-phenol resin; and a xylene resin; to produce a rubber-based adhesive. Above all, a rubber-based adhesive including a styrene-isoprene-styrene block copolymer as an adhesive base and a rosin ester-based resin as an adhesion imparting resin is particularly preferred from the viewpoint of the content stability and the suppression of the yellowing. In this case, the amount of the rosin ester-based adhesion imparting resin is preferably 2 to 50% by weight, based on the weight of the styrene-isoprene-styrene-based adhesive base.

In the adhesive, if required, a softener, such as a liquid polybutene, a liquid polyisobutylene and a mineral oil; a filler, such as titanium oxide and zinc oxide; an antioxidant, such as a hydrogen sulfite, a sulfite, a pyrosulfite, propyl gallate; can be incorporated. Above all, the incorporation of propyl gallate is particularly useful for enhancing the content stability and the suppression of the yellowing. The amount of propyl gallate is in the range of 0.01 to 10% by weight. When the amount is more than this range, the adhesive properties are adversely affected and when the amount is less than this range, the effect of suppressing the yellowing is lowered.

The percutaneous absorption formulation of the present invention contains ketotifen fumarate as an active ingredient in the adhesive layer. The amount thereof is usually 0.1 to 30% by weight, preferably 0.3 to 20% by weight. When the amount is more than this range, a crystal of ketotifen fumarate is separated out and the adhesion force of the formulation may be lowered. On the other hand, when the amount is less than this range, it becomes difficult to obtain persistently a satisfactory drug efficacy.

Further, the percutaneous absorption formulation of the present invention contains besides ketotifen fumarate, tris(hydroxymethyl)aminomethane (Tris) in the adhesive layer. Examples of a basic substance conventionally used for isolating an active ingredient in a salt form in a formulation include many substances, such as potassium hydroxide, sodium hydroxide, calcium hydroxide, magnesium hydroxide, sodium carbonate, sodium hydrogen carbonate, a phosphate, a borate, an acetate, ammonia, monoethanol amine, diethanol amine, triethanol amine and diisopropanol amine. However, among them, sodium carbonate, sodium hydrogen carbonate, a phosphate and the like are unlikely to be dissolved in an organic solvent, so that they are unsuitable for the use in a solvent-based adhesive. Further, potassium hydroxide and sodium hydroxide are soluble in an alcohol and consequently, can be used in a solvent-based adhesive. However, these compounds have high deliquescency and are easily reacted with carbon dioxide in air, so that they are difficult to handle. Further, monoethanol amine, diethanol amine, triethanol amine and diisopropanol amine may be yellowed by oxygen, heat, light or the like even after a neutralization reaction, so that they have a danger of accelerating the yellowing of the formulation.

On the contrary, Tris used in the present invention is a crystalline substance having a low hygroscopic property and is stable to light and heat, so that it can be easily handled. Particularly when Tris is incorporated in the adhesive layer together with ketotifen fumarate, it can improve the skin permeability of ketotifen fumarate and can extremely suppress the yellowing of the formulation. The amount of Tris is usually 20 to 100% by weight, preferably 25 to 70% by weight, based on the weight of ketotifen fumarate. When the amount is more than this range, a stimulation of the skin by the formulation may be caused. On the other hand, when the amount is less than this range, satisfactory skin permeability of the formulation cannot be obtained. Tris can be incorporated in the adhesive in the form of a crystal or powder as is. However, it is also possible that Tris in the form of a crystal or powder is dissolved or dispersed in an appropriate organic solvent and the resultant solution or dispersion is incorporated in the adhesive.

In the percutaneous absorption formulation of the present invention, for enhancing the percutaneous absorption of ketotifen fumarate, if desired, a percutaneous absorption enhancer can be incorporated in the adhesive layer. In the present invention, the percutaneous absorption enhancer enhances not only the percutaneous absorption of a drug, but also the skin permeation of a drug, so that it refers to also as a skin permeation enhancer. Examples of the percutaneous absorption enhancer include, but not limited to, an aliphatic alcohol, an aliphatic acid, an aliphatic acid ester, a polyhydric alcohol alkyl ether, a polyoxyethylene alkyl ether, a glyceride (an aliphatic acid ester of glycerin), a polyhydric alcohol-middle chain aliphatic acid ester, an alkyl lactate ester, a dibasic acid alkyl ester, an acylated amino acid and pyrrolidones. These percutaneous absorption enhancers can be used individually or in combination of two or more.

Preferred examples of the aliphatic alcohol include (12 to 22 C) saturated or unsaturated higher alcohols, such as oleyl alcohol and lauryl alcohol.
Examples of the aliphatic acid include linoleic acid, oleic acid, linolenic acid, stearic acid, isostearic acid and palmitic acid.
Examples of the aliphatic acid ester include isopropyl myristate, diisopropyl adipate and isopropyl palmitate.
Examples of the polyhydric alcohol alkyl ether include alkyl ethers of polyhydric alcohols, such as glycerin, ethylene glycol, propylene glycol, 1,3-butylene glycol, diglycerin, polyglycerin, diethylene glycol, polyethylene glycol, dipropylene glycol, polypropylene glycol, sorbitan, sorbitol, isosorbide, methyl glucoside, oligosaccharide and reduced oligosaccharide. The carbon number of an alkyl part of the polyhydric alcohol alkyl ether is preferably 6 to 20.
As the polyoxyethylene alkyl ether, preferred is that in which the carbon number of an alkyl part thereof is 6 to 20 and the number of recurring units (-O-CH₂CH₂-) of the polyoxyethylene chain is 1 to 9. Specific examples thereof include polyoxyethylene lauryl ether, polyoxyethylene cetyl ether, polyoxyethylene stearyl ether and polyoxyethylene oleyl ether.
As the glyceride, a glycerin ester of a (6 to 18 C) aliphatic acid is preferably used. The glyceride is classified into monoglyceride, diglyceride and triglyceride according to the number of bonded aliphatic acids and all of them can be used in the present invention. Also, a mixture thereof, such as a mixture of monoglyceride and diglyceride, can be also used. Examples of aliphatic acids forming the glyceride include octanoic acid, decanoic acid, dodecanoic acid, tetradecanoic acid, hexadecanoic acid, octadecanoic acid (stearic acid) and oleic acid.
Examples of other percutaneous absorption enhancers include lactic acid, tartaric acid, 1,2,6-hexanetriol, benzyl alcohol and lanolin.

Above all, an aliphatic higher alcohol, such as lauryl alcohol; an aliphatic acid, such as isostearic acid; an aliphatic acid ester, such as isopropyl miristate and isopropyl palmitate; a polyoxyethylene alkyl ether, such as polyoxyethylene oleyl ether; and a mixture of two or more of the foregoing are preferred. The percutaneous absorption enhancer is used in an amount of usually 1 to 50% by weight, preferably 2 to 40% by weight.

As the substrate, preferred is a substrate which adsorbs no agent in the adhesive layer and from which no agent is released. Specific examples thereof include a nonwoven fabric, a woven fabric, a film or sheet, a porous material, a foam, paper and a complex produced by laminating these materials.

Examples of a material for the nonwoven fabric include a polyolefinic resin, such as a polyethylene and a polypropylene; a polyester resin, such as a polyethylene terephthalate, a polybutylene terephthalate and a polyethylene naphthalate; rayon; a polyamide; a polyester ether; a polyurethane; a polyacrylic resin; a polyvinyl alcohol; a styrene-isoprene-styrene copolymer; and a styrene-ethylene-propylene-styrene copolymer.
Examples of a material for the woven fabric include cotton, rayon, a polyacrylic resin, a polyester resin and a polyvinyl alcohol.
Examples of a material for the film or sheet include a polyolefinic resin, such as a polyethylene and a polypropylene; a polyacrylic resin, such as a polymethyl methacrylate and a polyethylene methacrylate; a polyester resin, such as a polyethylene terephthalate, a polybutylene terephthalate and a polyethylene naphthalate; a cellophane; a polyvinyl alcohol; an ethylene-vinyl alcohol copolymer; a polyvinyl chloride; a polystyrene; a polyurethane; a polyacrylonitrile; a fluorine resin; a styrene-isoprene-styrene copolymer; a styrene-butadiene rubber; a polybutadiene; an ethylene-vinyl acetate copolymer; a polyamide; and a polysulfone.
Examples of the paper include impregnated paper, coated paper, quality paper, craft paper, Japanese paper, glassine paper and synthetic paper.
Examples of the complex include a complex produced by laminating the above film or sheet on the above nonwoven fabric or woven fabric.

The patch in the percutaneous absorption formulation of the present invention can be produced by disposing the adhesive layer containing an adhesive on the substrate by a coating method, a hot melt method, a calendar method or the like.

In the coating method, the patch is produced, for example by coating an organic solution containing ketotifen fumarate, Tris, and other additives and an adhesive on a substrate or a release liner and by drying the resultant coating. Examples of a solvent of the organic solution include toluene, ethyl acetate and hexane.
In the hot melt method, the patch is produced, for example by: melting an adhesive component by heating and stirring it under purging with nitrogen; adding ketotifen fumarate, Tris and other additives to the above-molten adhesive component after lowering the reaction temperature, and mixing the resultant mixture homogeneously; spreading the mixture on a release liner with a hot melt coater; and laminating a substrate.
In the calendar method, the patch is produced, for example by: masticating an adhesive base; adding an adhesion imparting resin to the adhesive base after lowering the reaction temperature, and kneading the resultant mixture; adding a softener to the mixture after further lowering the reaction temperature, and kneading the resultant mixture; adding finally ketotifen fumarate, Tris and other additives to the mixture and kneading the resultant mixture; spreading the resultant mixture on a release liner; and laminating a substrate.
In these production methods, the reaction temperature and the kneading time can be appropriately varied according to the formulation of the adhesive, or the like. Further, the adhesive layer has a thickness of usually 10 to 300 µm.

In the coating method, when it is necessary to cause ketotifen fumarate to be more easily dissolved in an adhesive, if desired, a solubilizer can be used. Specific examples thereof include crotamiton, ethanol, urea, an aliphatic acid ester of propylene glycol, 1-menthol and mentha oil. These solubilizers can be used individually or in combination of two or more. Further, the solubilizer is used in an amount of usually 0 to 40% by weight, preferably 0 to 20% by weight.

In the hot melt method or the calendar method, usually a mixture containing various components and an adhesive is spread on a release liner. If desired, the mixture is spread on a substrate and thereon, a release liner can be disposed as a coating agent.

Examples of the packaging material having a hygroscopic property used in the present invention include a hygroscopic packaging material containing a hygroscopic substance and a packaging material in which an absorbent is held in a moisture impermeable packaging material. To avoid problems of a high bulkiness of the formulation and drinking of the absorbent by mistake, it is preferred that a hygroscopic packaging material be used. Further, more preferred is a packaging material having light blocking effects from the viewpoint of the content stability and the suppression of the yellowing, or a packaging material having heat-sealing properties from the viewpoint of packaging workability.

Examples of the packaging material include materials produced by laminating one or more types of resin layers having heat-sealing properties, made of for example a polyethylene, a polypropylene or ethylene vinyl acetate, on a foil of a metal, such as aluminum. On the outer surface of the aluminum or other metal foil, a polyester, cellophane or paper, etc. may be further laminated.

While the percutaneous absorption formulation of the present invention includes the patch packaged in the hygroscopic packaging material, it is preferred that an atmosphere of the inside of the packaging material having already packaged the patch contain substantially no oxygen. The removal of oxygen from the atmosphere can be performed by imparting a deoxidation function to the packaging material itself, by causing the oxygen impermeable packaging material to hold a deoxidizing agent, or by purging the atmosphere in the inside of the packaging material with nitrogen. Thus, the effect of the content stability and of the suppression of the yellowing is further improved.

Hereinafter, the present invention will be more specifically described referring to examples which should not be construed as limiting the scope of the present invention. Hereinafter, unless indicated otherwise, "%" means "% by mass".

### Example 1

Among the components shown in Table 1, an SIS-based adhesive base: Quintac 3570 C (trade name; manufactured by ZEON Corporation), a rosin ester-based adhesion imparting resin: Pine Crystal KE 311 (trade name; manufactured by Arakawa Chemical Industries, Ltd.) and isopropyl palmitate were dissolved in toluene and to the resultant solution, ketotifen fumarate and Tris were added to obtain an adhesive. The obtained adhesive was coated on a PET film having a thickness of 75 µm which had been subjected to a silicone treatment so that the resultant dried adhesive layer had a thickness of 40 µm and the PET film was dried at 110°C for 3 minutes to thereby provide an adhesive layer. Next, on the adhesive layer, a PET film having a thickness of 25 µm was laminated. The thus obtained patch was packaged in a hydroscopic packaging material (trade name: Toyal Dry; manufactured by Toyo Aluminum K.K.) to thereby obtain a percutaneous absorption formulation of the present invention.

### Example 2

In substantially the same manner as in Example 1, except that the atmosphere in the inside of the packaging material was purged with nitrogen (purged rate: 70% or more), a percutaneous absorption formulation of the present invention was obtained.

### Example 3

In substantially the same manner as in Example 1, except that in the adhesive, further a 10% solution (methanol/toluene = 1/9) of propyl gallate was incorporated, a percutaneous absorption formulation of the present invention was obtained.

### Example 4

In substantially the same manner as in Example 3, except that the atmosphere in the inside of the packaging material was purged with nitrogen (purged rate: 70% or more), a percutaneous absorption formulation of the present invention was obtained.

### Comparative Example 1

In substantially the same manner as in Example 1, except that as a basic substance, instead of Tris, monoethanol amine was used; as an antioxidant, dibutylhydroxy toluene (BHT) was incorporated; and the patch was not packaged in a hygroscopic packaging material, a percutaneous absorption formulation as a comparative example was obtained.

### Comparative Example 2

In substantially the same manner as in Comparative Example 1, except that as an adhesion imparting resin, a terepene-based adhesion imparting resin: YS resin PX-1150N (trade name; manufactured by Yasuhara Chemical Co., Ltd) was used, a percutaneous absorption formulation as a comparative example was obtained.

### Comparative Example 4

In substantially the same manner as in Comparative Example 1, except that as an adhesion imparting resin, a petroleum alicyclic adhesion imparting resin: ARKON P-100 (trade name; manufactured by Arakawa Chemical Industries, Ltd.), a percutaneous absorption formulation as a comparative example was obtained.

### Comparative Example 4

In substantially the same manner as in Example 1, except that the patch was not packaged in a hygroscopic packaging material, a percutaneous absorption formulation as a comparative example was obtained.

### Comparative Example 5

In substantially the same manner as in Example 3, except that the patch was not packaged in a hygroscopic packaging material, a percutaneous absorption formulation as a comparative example was obtained.

### Comparative Example 6

In substantially the same manner as in Example 2, except that as a basic substance, instead of Tris, monoethanol amine was used; and as an antioxidant, dibutylhydroxy toluene (BHT) was incorporated, a percutaneous absorption formulation as a comparative example was obtained.

**[Table 1] Formulation (in % by weight) of Adhesive, and Presence of Hygroscopic Packaging Material and Purge with Nitrogen**

| | Examples | | | | Comparative Examples | | | | | |
|---|---|---|---|---|---|---|---|---|---|---|
| | 1 | 2 | 3 | 4 | 1 | 2 | 3 | 4 | 5 | 6 |
| SIS-based Adhesive Base | 37.5 | 37.5 | 37.4 | 37.4 | 38.4 | 38.4 | 38.4 | 37.5 | 37.4 | 38.4 |
| Adhesion Imparting Resin 1 | 37.5 | 37.5 | 37.4 | 37.4 | 38.4 | - | - | 37.5 | 37.4 | 38.4 |
| Adhesion Imparting Resin 2 | - | - | - | - | - | 38.4 | - | - | - | - |
| Adhesion Imparting Resin 3 | - | - | - | - | - | - | 38.4 | - | - | - |
| Isopropyl Palmitate | 10.0 | 10.0 | 10.0 | 10.0 | 10.0 | 10.0 | 10.0 | 10.0 | 10.0 | 10.0 |
| Ketotifen Fumarate | 10.0 | 10.0 | 10.0 | 10.0 | 10.0 | 10.0 | 10.0 | 10.0 | 10.0 | 10.0 |
| Tris | 5.0 | 5.0 | 5.0 | 5.0 | - | - | - | 5.0 | 5.0 | - |
| Monoethanol Amine | - | - | - | - | 2.5 | 2.5 | 2.5 | - | - | 2.5 |
| Propyl Gallate | - | - | 0.2 | 0.2 | - | - | - | - | 0.2 | - |
| BHT | - | - | - | - | 0.7 | 0.7 | 0.7 | - | - | 0.7 |
| Hygroscopic Packaging Material | YES | YES | YES | YES | NO | NO | NO | NO | NO | YES |
| Purging with Nitrogen | NO | YES | NO | YES | NO | NO | NO | NO | NO | YES |

| | | | | | | | | | | |
|---|---|---|---|---|---|---|---|---|---|---|
| Adhesion Imparting Resin 1: Pine Crystal KE311 (manufactured by Arakawa Chemical Industries, Ltd.) Adhesion Imparting Resin 2: YS resin PX-1150N (manufactured by Yasuhara Chemical Co., Ltd.) Adhesion Imparting Resin 3: ARCON P-100 (manufactured by Arakawa Chemical Industries, Ltd.) | | | | | | | | | | |

With respect to the percutaneous absorption formulations in Examples 1 to 4 and Comparative Examples 1 to 6, the content stability and the suppression of the yellowing after the formulations were preserved at 40°C and in 75% relative humidity for 6 months were evaluated.
The content stability was expressed in the percentage (%) of the concentration of ketotifen fumarate in the patch after 6 months to the concentration (initial value) of ketotifen fumarate in the patch immediately after the production of the patch, wherein the concentration of ketotifen fumarate was measured by a liquid chromatography. The patch in which the concentration of ketotifen fumarate remained at 95% or more relative to the initial value, was evaluated as "A", at 90% or more to less than 95% was evaluated as "B", and at less than 90% was evaluated as "C".
The suppression of the yellowing was expressed in ΔYI value (YI value after 6 months - YI value immediately after the production of the patch), wherein the YI value was obtained by measuring the color tone of the patch using a color computer (manufactured by Suga Test Instruments Co., Ltd.). The patch in which ΔYI value was less than 30 was evaluated as "A" and 30 or more was evaluated as "B".
The results are shown in Table 2.

**[Table 2] Measurement Results**

| | Content Stability | | Suppression of Yellowing | |
|---|---|---|---|---|
| | % Relative to initial value | Evaluation | ΔYI | Evaluation |
| Example 1 | 95.6 | A | 27.6 | A |
| Example 2 | 99.7 | A | 15.1 | A |
| Example 3 | 97.7 | A | 23.1 | A |
| Example 4 | 99.0 | A | 11.2 | A |
| Comparative Example 1 | 80.1 | C | 46.6 | B |
| Comparative Example 2 | 37.6 | C | 56.8 | B |
| Comparative Example 3 | 60.9 | C | 51.5 | B |
| Comparative Example 4 | 77.3 | C | 29.1 | A |
| Comparative Example 5 | 85.2 | C | 21.3 | A |
| Comparative Example 6 | 91.3 | B | 35.3 | B |

As apparent from the results shown in Table 2, in the percutaneous absorption formulation according to the present invention, the decreasing of the drug content and the yellowing were slight. Above all, Example 2 in which the atmosphere in the packaging material was purged with nitrogen and Example 3 in which propyl gallate was further incorporated, were more excellent in both the content stability and the suppression of the yellowing than Example 1. Example 4 in which the purge with nitrogen and the incorporation of propyl gallate were simultaneously performed exhibited the highest effect.
On the contrary, in Comparative Examples 1 to 3, since monoethanol amine was used as a basic substance instead of Tris, they were poor in the content stability, which was not improved even by using other adhesion imparting resins. In comparison with Comparative Example 2 in which a terepene-based adhesion imparting resin was used and with Comparative Example 3 in which an alicyclic adhesion imparting resin was used, Comparative Example 1 in which a rosin ester-based adhesion imparting resin was used was more excellent in the content stability.
Further, in Comparative Example 4 in which no hygroscopic packaging material was used, the suppression of the yellowing was satisfactory, however, the content stability was problematic. Even in Comparative Example 5 in which propyl gallate was further incorporated, a satisfactory result could not be obtained.
Further, as is shown by Comparative Example 6, even when the patch is packaged in a hygroscopic packaging material and the atmosphere in the inside of the packaging material is purged with nitrogen, when Tris is not used as a basic substance, satisfactory content stability and suppression of the yellowing could not be achieved.

## Claims

1. A percutaneous absorption formulation comprising:
a patch having an adhesive layer disposed on a substrate,
the adhesive layer comprising ketotifen fumarate and tris(hydroxymethyl) aminomethane, and
the patch being packaged in a hygroscopic packaging material.

2. The percutaneous absorption formulation according to claim 1, wherein the adhesive layer further contains 0.01 to 10% by weight of propyl gallate.

3. The percutaneous absorption formulation according to claim 1, wherein the adhesive layer comprises an SIS-based adhesive base and a rosin ester-based adhesion imparting resin.

4. The percutaneous absorption formulation according to claim 1, wherein the adhesive layer comprises an SIS-based adhesive base and a rosin ester-based adhesion imparting resin, and further contains 0.01 to 10% by weight of propyl gallate.

5. The percutaneous absorption formulation according to claim 1, wherein
the packaging material has a deoxidation function,
the packaging material is oxygen impermeable and a deoxidizing agent is packaged together therein, or
the inside of the packaging material is purged with nitrogen.
